# FASCICULE DE BREVET EUROPEEN

(11) **EP 0 698 616 B1**
(45) Date de publication et mention de la délivrance du brevet: **17.12.2003**
(21) Numéro de dépôt: 95401741.4
(22) Date de dépôt: 24.07.1995
(51) Int. Cl.: C07K 14/81

(54) **Procédé de préparation d'un concentré d'inhibiteur de la C1-estérase (C1-INH), et concentré obtenu, à usage thérapeutique**
Verfahren zur Herstellung eines C1-Esterase-Inhibitorkonzentrates (C1-INH) und Konzentrat zur therapeutischen Anwendung
Process for the preparation of a C1-esterase inhibitor (C1-1NH) concentrate and concentrate obtained by said process, suitable for therapeutical use

(30) Priorité: 28.07.1994 FR 9409353
(43) Date de publication de la demande: 28.02.1996
(73) Titulaire: ASSOCIATION POUR L'ESSOR DE LA TRANSFUSION SANGUINE DANS LA REGION DU NORD, 59000 Lille (FR)
(72) Inventeur: Poulle, Michel, F-59136 Wavrin (FR); Burnouf, née Radosevich, Miryana, F-59136 Wavrin (FR)
(74) Mandataire: Lepeudry-Gautherat, Thérèse

(56) Documents cités:
- US-A- 5 030 578
- JOURNAL OF CHROMATOGRAPHY, vol. 582, AMSTERDAM NL, pages 65-70, L-C TEH AND M FROGER 'Evaluation of the chromatographic procedure for the preparation of high-purity C1-esterase inhibitor concentrate from cryosupernatant plasma'
- JOURNAL OF CHROMATOGRAPHY, vol. 1, no. 510, pages 133-140, XP 000133540 MULLER W 'NEW ION EXCHANGERS FOR THE CHROMATOGRAPHY OF BIOPOLYMERS'
- BLOOD COAGULATION AND FIBRINOLYSIS, vol. 5, no. 4, Août 1994 pages 543-549, M. POULLE ET AL 'Large scale preparation of highly purified human C1-inhibitor for therapeutic use'

## Description

L'invention concerne un procédé de préparation d'un concentré d'inhibiteur de la C1-estérase, à partir d'une fraction de plasma humain, qui comprend 2 séparations par chromatographie sur gels échangeurs d'anions puis de cations et deux traitements d'élimination de contaminants viraux éventuels. Le concentré obtenu est destiné à un usage thérapeutique.

L'inhibiteur de la C1-estérase (connu sous l'abréviation C1-INH) est un inhibiteur de protéases présent dans le plasma qui contrôle l'activation du premier composant du complément, C1, et qui inactive ses sous-composants C1r et C1s. Il inhibe aussi d'autres sérine protéases de la coagulation et des systèmes fibrinolytiques et de contact, comme la kallicréine, le facteur Hageman activé par la plasmine et le facteur XIa.

Le C1-INH du plasma humain est une chaîne polypeptidique simple, glycosylée, d'un poids moléculaire de 104.000 daltons.

Des déficiences en C1-INH sont associées à des pathologies plus ou moins sévères pouvant même être létales. Des patients présentant un angioedème héréditaire sont dépourvus de C1-INH (type 1) ou présentent un C1-INH dont l'activité est réduite (type II). Une autre forme (type III) d'angioedème héréditaire est due à la liaison anormale de l'albumine au C1-INH. Il existe également des déficiences acquises en C1-INH provoquées par un catabolisme anormalement élevé de l'inhibiteur ou par la présence d'autoanticorps.

La maladie se manifeste par des oedèmes de la face et des extrémités, de la paroi intestinale et des voies respiratoires supérieures.

Divers traitements sont administrés à titre prophylactique pour réduire la sévérité des crises, avec des agents antifibrinolytiques ou des hormones, mais ceux-ci ne sont pas efficaces contre les crises aigües et, de plus, ils entraînent des effets secondaires incompatibles avec des traitements à long terme. Pour les crises aigües, des traitements aux inhibiteurs de kallicréine, comme l'aprotinine, sont appliqués mais ils peuvent entraîner des allergies.

Une thérapie de remplacement du C1-INH avec du plasma frais a déjà été pratiquée sur des patients à haut risque devant subir une intervention chirurgicale dentaire. Cette thérapie a entraîné une rémission des symptômes de la maladie. Cependant le plasma apporte un supplément de protéines inutiles, en particulier des constituants du complément C2 et C4, ce qui peut aggraver la pathologie.

La thérapie de remplacement avec du C1-INH purifié apparaît comme le traitement de choix pour les crises aigües comme pour la thérapie à long terme ou la prophylaxie préopératoire.

Actuellement quelques concentrés partiellement purifiés sont disponibles mais tous les procédés de préparation décrits sont difficiles à adapter à l'échelle industrielle, soit à cause de difficultés de mise en oeuvre, soit à cause du coût des matériaux à utiliser :
- Harrison (Biochemistry 1983; 22 : 5001-5007) a décrit un procédé de purification applicable à l'échelle du laboratoire, en vue de la caractérisation de la molécule, mais trop long pour être transposable à l'échelle industrielle ;
- Reboul et al. (Fed. Eur. Biochem. Soc. Lett. 1977; 79 : 45-50) et Pilatte et al. (J. Immunol. Methods 1989; 120 : 37-43) ont décrit des procédés de préparation comprenant une chromatographie d'affinité sur des lectines ;
- Prograis et al. (J. Immunol. Methods 1987; 99 : 113-122) ont décrit un procédé comprenant une chromatographie d'affinité sur gel chélaté au zinc ;
- Alsenz et al. (J. Immunol. Methods 1987; 96 : 107-114) ont décrit un procédé de préparation par immunoadsorption avec un anticorps monoclonal.

Dans tous ces procédés, des stabilisants toxiques sont additionnés aux tampons pour protéger l'activité du C1-INH.
- Un premier procédé de production à grande échelle a été décrit par Vogelaar et al. (Vox Sang. 1974; 26 : 118-127) par adsorption "en batch" sur DEAE-Sephadex® et élution à forte concentration en sel puis précipitation au sulfate d'ammonium pour éliminer l'albumine et la céruloplasmine.
- Le procédé a été amélioré (Wickerhauser. Joint Meet. 18th Congr. ISH/16th Congr. ISBT, Montreal 1980, Abst: 161 et Gadek et al. New Eng. J. Med. 1980; 302 : 542-546) par la combinaison de 2 chromatographies, sur DEAE-Sephadex® puis sur CM-Sephadex®, "en batch", et d'une précipitation au PEG, le produit final étant d'une "pureté intermédiaire".
- Enfin un produit "de haute pureté" a été obtenu en ajoutant une étape finale de précipitation en présence de sels (Wickerhauser. Vox Sang. 1987; 53: 1-6) ou en remplaçant le CM-Sephadex® par du CM-Sepharose®-FF (Benny et al. J. Chromatogr. 1988; 433 : 363-366). Fuhge et al. (Transfus. Sci. 1990, 11 : 23S-33S) ont aussi décrit un procédé comprenant deux chromatographies sur QAE- et phényl-Sepharose® et une précipitation au sulfate d'ammonium.

Ces divers procédés comprennent tous des étapes de précipitation qui doivent être suivies de centrifugation ; celles-ci sont peu sélectives, difficiles à mettre en oeuvre à grande échelle et ont des rendements inférieurs.

Pour contourner ces difficultés, la Demanderesse a réussi à mettre au point un procédé qui ne comprend pas d'étape de précipitation ou d'adsorption "en batch" mais seulement des chromatographies sur colonnes.

En outre la Demanderesse a testé différents types de gels de chromatographie et a choisi des gels échangeurs d'ions non conventionnels de type "tentaculaire" dont elle a mis en évidence la capacité de séparation particulièrement performante pour le C1-INH.

Les gels échangeurs d'ions tentaculaires sont décrits par Müller (J. Chromatography-1990; 510 : 133-140). Ils sont commercialisés par Merck (Darmstadt-Allemagne) sous l'appellation Fractogel-EMD® précédé de l'abréviation du groupement échangeur d'ions (TMAE-, DEAE-, DMAE-, SO₃-, COO-). Ils consistent en une matrice inerte, poreuse et semi-rigide, de type Fractogel® qui est constitué d'un copolymère d'oligoéthylèneglycol, glycidylméthacrylate et de pentaérythrol-diméthacrylate, sur laquelle sont greffés des tentacules constitués de chaînes de polymères vinyliques linéaires (et non réticulées comme dans les gels traditionnels). Les groupements échangeurs d'ions qui sont portés par ces chaînes linéaires ont une plus grande mobilité et sont plus indépendants de la matrice que dans les configurations réticulées courantes. Ces propriétés justifient le nom de gels à bras mobiles ou tentaculaires.

Ainsi le procédé selon la présente invention comprend deux étapes de séparation sur colonnes de chromatographie avec des gels échangeurs d'ions de type tentaculaire, la première sur gel échangeur faible d'anions, la seconde sur gel échangeur fort de cations.

L'échangeur faible d'anions est de type DMAE-, le polymère vinylique greffé sur la matrice étant constitué de monomères de formule CH₂ = CHCONH(CH₂)₂N(CH₃)₂·

L'échangeur fort de cations est un groupement SO₃⁻, le polymère vinylique greffé sur la matrice étant constitué de monomères de formule CH₂ = CHCONHC(CH₃)₂CH₂SO₃⁻.

Pour mettre en oeuvre le procédé de l'invention, on utilise comme matériau de départ une fraction de plasma humain dépourvue du cryoprécipité et prépurifiée par adsorption sur un gel greffé de groupements DEAE-, de type DEAE-Sephadex®, ce qui élimine les protéines dépendantes de la vitamine K, puis par une chromatographie d'affinité sur héparine immobilisée, ce qui élimine l'antithrombine III.

Ainsi le procédé n'interfère pas avec le fractionnement et la préparation des autres facteurs sanguins d'intérêt thérapeutique.

Le procédé selon l'invention comprend les étapes suivantes :
- charge de la fraction de plasma prépurifiée sur la première colonne de chromatographie,
- séparation chromatographique sur le gel faiblement échangeur d'anions et élimination du filtrat qui contient l'albumine, les IgG, les IgA, l'α.AT et l'α₂-macroglobuline,
- élution et élimination du constituant C3 du complément,
- élution et récupération de la fraction contenant le C1-INH,
- charge de l'éluat sur la seconde colonne de chromatographie,
- séparation chromatographique sur le gel fortement échangeur de cations et élimination du filtrat et d'une première fraction contenant des IgM,
- élution et récupération du C1-INH,
- concentration et lyophilisation de l'éluat.

La première séparation chromatographique est réalisée en tampon phosphate de sodium 20mM, pH 7, additionné de NaCl 60 mM. L'augmentation de la concentration en NaCl du tampon à 120 mM permet d'éluer les protéines indésirables, en particulier le constituant C3 du complément ; ensuite l'augmentation de la concentration en NaCl à 170 mM permet d'éluer le C1-INH.

Là seconde séparation chromatographique est réalisée en tampon phosphate de sodium 20 mM, pH 7, additionné de NaCl 30 mM et l'élution du C1-INH est obtenue par augmentation de la concentration en NaCl du tampon à 90 mM. Une première fraction mineure, contenant des IgM, s'élue dans les mêmes conditions et est éliminée ; la fraction majeure contenant le C1-INH sort de la colonne bien séparée de la première.

Le procédé selon l'invention comprend également, de préférence, 2 traitements d'inactivation virale. Le premier, par traitement au solvant/détergent, est appliqué à l'éluat de la première chromatographie de telle sorte que les agents utilisés soient éliminés dans le filtrat de la chromatographie suivante. Le second traitement est une nanofiltration séquentielle sur filtres de 35 puis 15 nm, qui est destinée à éliminer d'éventuels contaminants viraux de petite taille. Celle-ci est effectuée sur l'éluat concentré de la seconde chromatographie.

L'invention couvre également le concentré de C1-INH obtenu par le procédé décrit plus haut, et qui présente une activité spécifique au moins égale à 6 U/mg protéine, correspondant à une pureté supérieure à 85 %. Son degré de pureté, ses caractéristiques biochimiques et son activité biologique le rendent tout à fait acceptable pour un usage thérapeutique, non seulement pour compenser les déficiences en C1-INH décrites plus haut mais aussi pour traiter diverses pathologies dans lesquelles des réactions inflammatoires sont catalysées par la kallicréine et le facteur de Hageman activé.

Les exemples suivants illustrent l'invention sans en limiter la portée.

### Exemple 1 Purification du C1-INH

Une fraction de plasma humain dépourvue du cryoprécipité est soumise à 2 étapes de prépurification
- par adsorption sur DEAE-Sephadex®A50 (Pharmacia-LKB) pour éliminer les protéines dépendantes de la vitamine K
- et par chromatographie d'affinité sur héparine-Sepharose® (Pharmacia-LKB) pour éliminer l'antithrombine III.

Le filtrat de la chromatographie sur héparine-Sepharose contient 50 à 70 % de la concentration normale de C1-INH du plasma qui est en moyenne de 0,24 g/l.

Les étapes de purification sont réalisées à température ambiante, par lots de 180 litres de fraction prépurifiée.

Après dialyse sur un système d'ultrafiltration 10 K (Filtron®) contre le tampon d'équilibrage de la chromatographie, la fraction de plasma est soumise à une chromatographie d'échange d'anions. La fraction de plasma est chargée sur une colonne de chromatographie Moduline® P 630-250 (Amicon) remplie de DMAE-Fractogel EMD® 650 M (Merck) équilibrée en tampon phosphate de sodium 20 mM, à pH 7, additionné de NaCl 60 mM.

Le gel est lavé avec le tampon d'équilibrage jusqu'à ce que l'absorbance du filtrat soit retombée à la ligne de base. Le filtrat de cette chromatographie contient l'albumine, les IgG, les IgA, l'α-AT et l'α₂-macroglobuline, qui sont recyclés dans le circuit du fractionnement. Ensuite le tampon est additionné de NaCl jusqu'à une concentration de 120 mM pour éliminer les protéines indésirables, en particulier le constituant C3 du complément.

Le C1-INH est ensuite élué par augmentation de la concentration en NaCl du tampon à 170 mM.

Les IgM, la céruloplasmine et le constituant C4 du complément restent fortement adsorbés sur la colonne.

L'éluat présente une activité spécifique de 1,3 ± 0,2 U C1-INH/mg protéine, correspondant à une pureté d'environ 30 %. L'éluat est ensuite concentré à 10 g protéines/l et dialysé (sur cassettes 10 K, Filtron®) contre le tampon d'équilibrage de la chromatographie suivante (SO₃-Fractogel).

L'éluat est ensuite soumis à un traitement d'inactivation virale au solvant-détergent, par mélange avec du TnBP 0,3 % et du Tween 80, 1 % (en concentrations finales) et incubation à 25°C pendant 7 heures sous agitation douce.

La solution est ensuite soumise à une chromatographie d'échange de cations sur SO3-Fractogel EMD®.

La solution traitée au solvant-détergent est chargée sur une colonne Bioprocess® 252 (Pharmacia) remplie de SO₃-Fractogel EMD® 650(M) (Merck) équilibrée en tampon phosphate de sodium 20 mM, à pH 7, additionné de NaCl 30 mM.

Le gel est lavé avec au moins 12 fois le volume de la colonne du même tampon pour éliminer les agents d'inactivation virale.

Le C1-INH est ensuite élué par augmentation de la concentration en NaCl du tampon à 90 mM.

Une première fraction contenant essentiellement des IgM est éliminée. La fraction principale contenant le C1-INH, s'élue ensuite, nettement séparée de la première, et elle est récupérée. Elle présente une activité spécifique de 6,5 ± 0,5 U/mg de protéine correspondant à une pureté de 84 à 92 %.

Le taux de récupération du produit purifié est de 40 à 50 % à partir de la fraction de départ et de 25 à 30 % à partir du plasma total.

L'éluat est ensuite concentré et dialysé (sur cassettes 30 K, Filtron®) en tampon citrate trisodique 10 mM à pH 7, additionné de NaCl 150 mM.

Les 2 types de colonnes sont régénérées par lavages successifs avec du NaCl 1 M, de l'hydroxyde de sodium 1 M et du NaCl 2 M.

L'éluat concentré (1,2 litre à environ 10 g/l d'antigène C1-INH) est soumis à un second traitement d'élimination de virus par nanofiltration séquentielle sur des filtres de porosité de 35 puis de 15 nm (Asahi BMM) . L'efficacité de ce traitement pour l'élimination de virus marqueurs de petite taille comme le virus de la poliomyélite et le parvovirus bovin a déjà été démontrée.

Le concentré est ensuite distribué en flacons (5 ml/flacon) et lyophilisé.

### Exemple 2 Analyse du produit obtenu

L'activité C1-INH est mesurée comme la capacité d'inhiber la C1-estérase avec un substrat chromogénique (Berichrom®). Une unité (UI) d'activité C1-INH est définie comme l'activité présente dans 1 ml de plasma normal.

Le produit final lyophilisé est reconstitué en solution instantanément (<1 minute) dans l'eau PPI ; la solution est claire, incolore et stable pendant 24 heures à température ambiante.

Les caractéristiques du produit final apparaissent dans le tableau suivant.

| | |
|---|---|
| Protéines totales, g/l | 10,5 ± 1,5 |
| C1-INH-antigène, g/l | 9 ± 2 |
| C1-INH-activité, U/ml | 70 ± 10 |
| Activité spécifique, U/mg protéine | 6,5 ± 0,5 |
| C3, g/l | 0,59 ± 0,29 |
| C4, g/l | <0,006 |
| IgG, g/l | 0,041 ± 0,004 |
| IgA, g/l | 0,056 ± 0,006 |
| IgM, g/l | 0,14 ± 0,03 |
| Albumine, g/l | 0-0,03 |
| Fibrinogène, g/l | 0,071 ± 0,005 |
| Céruloplasmine, g/l | <0,015 |
| Tween 80, ppm | <10 |
| TnBP, ppm | 0,2 |

Le rapport entre l'activité du C1-INH et la quantité d'antigène-C1-INH, exprimés en U/ml, est de 1,7 à 2 ce qui indique que l'inhibiteur reste stable tout au long du procédé de purification.

Le principal contaminant est le constituant C3 du complément qui représente 4 à 10 % des protéines totales.

Les tests sur animaux montrent que le produit est apyrogène et ne présente pas de toxicité sur souris (la dose tolérée est supérieure à 2.000 U/kg de poids corporel de souris). L'injection intraveineuse à des rats, à des doses allant jusqu'à 300 U/kg de poids corporel, ne provoque ni hypotension ni modification du rythme cardiaque.

## Revendications

1. Procédé de préparation d'un concentré d'inhibiteur de la C1-estérase, C1-INH, à usage thérapeutique, **caractérisé en ce qu'**il comprend deux étapes de séparation sur colonnes de chromatographie, avec des gels échangeurs d'ions de type tentaculaire, la première sur gel échangeur faible d'anions, la seconde sur gel échangeur fort de cations.

2. Procédé selon la revendication 1, **caractérisé en ce que** les gels sont constitués d'une matrice poreuse inerte, de type copolymère d'oligoéthylèneglycol, glycidylméthacrylate et de pentaérythrol-diméthacrylate, greffée de polymères vinyliques linéaires non réticulés (tentacules) qui portent les groupements ioniques.

3. Procédé selon la revendication 1 ou 2, **caractérisé en ce que** l'échangeur faible d'anions est de type DMAE-.

4. Procédé selon l'une quelconque des revendications 1 à 3, **caractérisé en ce que** l'échangeur fort de cations est un groupement SO₃⁻.

5. Procédé selon l'une quelconque des revendications 1 à 4, **caractérisé en ce que** le matériau de départ est une fraction de plasma humain dépourvue du cryoprécipité et prépurifiée par adsorption sur un gel greffé de groupements DEAE- puis par chromatographie d'affinité sur héparine immobilisée.

6. Procédé selon l'une quelconque des revendications 1 à 5, **caractérisé en ce qu'**il comprend les étapes suivantes :
- charge de la fraction de plasma prépurifiée, selon la revendication 5, sur la première colonne de chromatographie,
- séparation chromatographique sur gel échangeur faible d'anions, selon la revendication 3, et élimination du filtrat,
- élution et élimination du C3 du complément,
- élution et récupération du C1-INH,
- charge de l'éluat sur la seconde colonne de chromatographie,
- séparation chromatographique sur gel échangeur fort de cations, selon la revendication 4, et élimination du filtrat et d'une première fraction contenant des IgM,
- élution et récupération du C1-INH,
- concentration et lyophilisation de l'éluat.

7. Procédé selon la revendication 6, **caractérisé en ce que** la première séparation chromatographique est réalisée en tampon phosphate de sodium 20 mM, pH 7, additionné de NaCl 60 mM et l'élution du C3 est obtenue par augmentation de la concentration en NaCl du tampon à 120 mM et celle du C1-INH, par augmentation de la concentration en NaCl du tampon à 170 mM.

8. Procédé selon la revendication 6, **caractérisé en ce que** la seconde séparation chromatographique est réalisée en tampon phosphate de sodium 20 mM, pH 7, additionné de NaCl 30 mM et l'élution du C1-INH est obtenue par augmentation de la concentration en NaCl du tampon à 90 mM.

9. Procédé selon l'une quelconque des revendications 1 à 8, **caractérisé en ce qu'**il comprend un traitement d'inactivation virale par solvant/détergent, sur l'éluat de la première chromatographie.

10. Procédé selon l'une quelconque des revendications 1 à 9, **caractérisé en ce qu'**il comprend une étape d'élimination d'éventuels contaminants viraux de petite taille par nanofiltration de l'éluat concentré de la seconde chromatographie.

11. Concentré de C1-INH à usage thérapeutique susceptible d'être obtenu par le procédé selon l'une quelconque des revendications 1 à 10, **caractérisé en ce qu'**il présente une activité spécifique au moins égale à 6 U/mg de protéine, correspondant à une pureté supérieure à 85 %.

12. Concentré de C1-INH selon la revendication 11, doublement viro-inactivé, **caractérisé en ce qu'**il a fait l'objet d'un traitement au solvant-détergent et par nanofiltration.

## Claims

1. A process for preparing a C1-esterase inhibitor, C1-INH, concentrate for therapeutic use, **characterised in that** it comprises two separation steps on chromatographic columns packed with tentacular type ion exchanger gels, the first one being performed on a weak anion exchanger gel, the second one on a strong cation exchanger gel.

2. A process according to claim 1, wherein said gels are constituted of an inert porous matrix, of a copolymer of oligoethyleneglycol, glycidylmethacrylate and pentaerythroldimethacrylate type with grafted non crosslinked linear vinyl polymers (tentacules) carrying ionic groupings.

3. A process according to claims 1 or 2, wherein the weak anion exchanger is of DMAE-type.

4. A process according to any one of claims 1 to 3, wherein the strong cation exchanger is a SO₃-grouping.

5. A process according to any one of claims 1 to 4, wherein the starting material is a human plasma fraction free of cryoprecipitate and pre-purified by adsorption on a gel with grafted DEAE-groupings, then by affinity chromatography on immobilised heparin.

6. A process according to any one of claims 1 to 5, comprising the following steps :
- loading the prepurified plasma fraction, according to claim 5, on the first chromatographic column,
- chromatographic separation on a weak anion exchanger gel according to claim 3, and elimination of the filtrate,
- elution and elimination of the complement component C3,
- elution and recovery of C1-INH,
- loading the eluate on the second chromatographic column,
- chromatographic separation on a strong cation exchanger gel according to claim 4 and elimination of the filtrate and of a first fraction containing IgM,
- elution and recovery of C1-INH,
- concentration and freeze-drying of the eluate.

7. A process according to claim 6, wherein the first chromatographic separation is carried out in 20 mM sodium phosphate buffer, pH 7, supplemented with 60 mM NaCl and the elution of the C3 is obtained by raising the NaCl concentration of the buffer to 120 mM and that of C1-INH, by raising the NaCl concentration of the buffer to 170 mM.

8. A process according to claim 6, wherein the second chromatographic separation is carried out in 20 mM sodium phosphate buffer, pH 7, supplemented with 30 mM NaCl and the elution of C1-INH is obtained by raising the concentration of NaCl of the buffer to 90 mM.

9. A process according to any one of claims 1 to 8, wherein the eluate of the first chromatography is submitted to a viral inactivation treatment with solvent/detergent.

10. A process according to any one of claims 1 to 9, comprising a step of elimination of possible small size viral contaminants by nanofiltration of the concentrated eluate of the second chromatography.

11. A C1-INH concentrate for therapeutic use obtainable by a process according to any one of claims 1 to 10, **characterised in that** it presents a specific activity at least equal to 6 U/mg of protein corresponding to a purity higher than 85 %.

12. A C1-INH concentrate according to claim 11, twice virally inactivated, resulting from a treatment with solvent/detergent and by nanofiltration.

## Patentansprüche

1. Verfahren zur Herstellung eines Konzentrats eines C1-Esteraseinhibitors (C1-INH) für die therapeutische Verwendung, **dadurch gekennzeichnet, dass** es zwei Trennstufen in chromatographischen Kolonnen umfasst, die Ionenaustauschergele vom beweglichen Fangarm-Typ enthalten, wobei die erste Stufe an einem schwachen Anionen-Austauschergel durchgeführt wird und die zweite Stufe an einem starken Kationen-Austauschergel durchgeführt wird.

2. Verfahren nach Anspruch 1, **dadurch gekennzeichnet, dass** die Gele bestehen aus einer inerten porösen Matrix vom Typ eines Copolymers von Oligoethylenglycol, Glycidylmethacrylat und Pentaerythrit-Dimethacrylat, auf das nichtvernetzte lineare Vinylpolymere (bewegliche Fangarme) aufgepfropft sind, welche die lonengruppen tragen.

3. Verfahren nach Anspruch 1 oder 2, **dadurch gekennzeichnet, dass** der schwache Anionenaustauscher ein solcher vom DMAE-Typ ist

4. Verfahren nach einem der Ansprüche 1 bis 3, **dadurch gekennzeichnet, dass** der starke Kationenaustauscher eine SO₃⁻-Gruppe ist.

5. Verfahren nach einem der Ansprüche 1 bis 4, **dadurch gekennzeichnet, dass** das Ausgangsmaterial eine Humanplasma-Fraktion ist, die frei von Cryopräzipitat ist und vorgereinigt worden ist durch Adsorption an einem Gel, auf das DEAE-Gruppen aufgepfropft sind, und anschließende Affinitäts-Chromatographie an immobilisiertem Heparin.

6. Verfahren nach einem der Ansprüche 1 bis 5, **dadurch gekennzeichnet, dass** es die folgenden Stufen umfasst:
- Aufgabe der vorgereinigten Plasmafraktion nach Anspruch 5 auf die erste Chromatographie-Kolonne,
- chromatographische Trennung an einem schwachen Anionen-Austauschergel nach Anspruch 3 und Eliminierung des Filtrats,
- Elution und Eliminierung von C3 des Komplements,
- Elution und Gewinnung von C1-INH,
- Aufgabe des Eluats auf die zweite Chromatographie-Kolonne,
- chromatographische Trennung an einem starken Kationen-Austauschergel nach Anspruch 4 und Eliminierung des Filtrats und einer ersten Fraktion, die IgM enthält,
- Elution und Gewinnung von C1-INH,
- Konzentration und Lyophilisierung des Eluats.

7. Verfahren nach Anspruch 6, **dadurch gekennzeichnet, dass** die erste chromatographische Trennung in einem 20 mM Natriumphosphat-Puffer von pH 7, versetzt mit 60 mM NaCl, durchgeführt wird und dass die Elution von C3 erhalten wird durch Erhöhung der NaCl-Konzentration des Puffers auf 120 mM und diejenige von C1-INH erhalten wird durch Erhöhung der NaCl-Konzentration des Puffers auf 170 mM.

8. Verfahren nach Anspruch 6, **dadurch gekennzeichnet, dass** die zweite chromatographische Trennung in einem 20 mM Natriumphosphat-Puffer von pH 7 durchgeführt wird, der mit 30 mM NaCl versetzt ist, und dass die Elution von C1-INH erhalten wird durch Erhöhung der NaCl-Konzentration des Puffers auf 90 mM.

9. Verfahren nach einem der Ansprüche 1 bis 8, **dadurch gekennzeichnet, dass** es eine Behandlung zur Vireninaktivierung durch ein Lösungsmittel-Detergens des Eluats der ersten Chromatographie umfasst.

10. Verfahren nach einem der Ansprüche 1 bis 9, **dadurch gekennzeichnet, dass** es eine Eliminierung von eventuellen viralen Verunreinigungen geringer Größe durch Nanofiltration des konzentrierten Eluats der zweiten Chromatographie umfasst.

11. C1-INH-Konzentrat für die therapeutische Verwendung, wie es nach dem Verfahren nach einem der Ansprüche 1 bis 10 erhältlich ist, **dadurch gekennzeichnet, dass** es eine spezifische Aktivität aufweist, die mindestens 6 U/mg Protein beträgt entsprechend einer Reinheit von mehr als 85 %.

12. C1-INH-Konzentrat nach Anspruch 11, das zweimal Virus-inaktiviert worden ist, **dadurch gekennzeichnet, dass** es einer Behandlung mit einem Lösungsmittel-Detergens und einer Nanofiltration unterworfen worden ist.
